# EUROPEAN PATENT APPLICATION

(11) **EP 4 509 535 A1**
(43) Date of publication of application: **19.02.2025**
(21) Application number: 23788253.5
(22) Date of filing: 06.04.2023
(51) Int. Cl.: C08F 10/14, A61K 8/31, A61Q 19/00, C10M 107/10

(54) **POLYOLEFIN, PLASTICIZER FOR ELECTRONIC MATERIAL, SUBSTRATE FOR COSMETIC PREPARATION, COSMETIC PREPARATION, AND LUBRICATING AGENT**

(30) Priority: 13.04.2022 JP 2022066549
(71) Applicant: NOF Corporation, Shibuya-ku Tokyo 150-6019 (JP)
(72) Inventor: SATO, Megumi, Kawasaki-shi, Kanagawa 210-0865 (JP); HONDA, Takuya, Kawasaki-shi, Kanagawa 210-0865 (JP); SEKIGUCHI, Koji, Kawasaki-shi, Kanagawa 210-0865 (JP); YOSHIKAWA, Fumitaka, Kawasaki-shi, Kanagawa 210-0865 (JP); SHIMIZU, Yutaro, Amagasaki-shi, Hyogo 660-0095 (JP)
(74) Representative: J A Kemp LLP
(86) International application number: PCT/JP2023/014201
(87) International publication number: WO 2023/199834

(57) **Abstract**

The present invention provides a polyolefin having a number average molecular weight of 250 to 10,000 and obtained from a linear olefin monomer having 8 to 20 carbon atoms, wherein a rate of side chains having (n-3) carbon atoms or lower than (n-3) carbon atoms is 1 to 40%, as calculated from the formula (1): rate of side chains having (n-3) carbon atoms or lower than (n-3) carbon atoms (%) = [Nx/(Nx+Ny)×2n-3]/3×100 [symbols in the formula (1) are as defined in the DESCRIPTION].

## Description

### [Technical Field]

The present invention relates to a novel polyolefin that can be used effectively in various applications such as plasticizer for electronic materials, base material for cosmetics, and the like. In addition, the present invention provides plasticizers for electronic materials, base materials for cosmetics, cosmetics containing the aforementioned base materials for cosmetics, and lubricants.

### [Background Art]

Polyolefin is a polymer made of olefins, represented by ethylene and propylene, as monomers. Polyolefin is a polymer that is composed only of C/H atoms, has no unsaturated bonds, and is characterized by low density, low polarity, high water resistance, and high chemical resistance. For this reason, polyolefins are used in various applications such as lubricant, plasticizer for electronic materials, base material for cosmetics, and the like.

Lubricants are used to reduce friction between mechanical elements of machines. In recent years, the environment where lubricants are used has become harsher, whereas longer life is tended to be desired in consideration of environmental issues. Thus, the demand for poly-α-olefin (PAO) synthetic lubricating oils, which are superior in low-temperature viscosity characteristics, heat resistance, and oxidation stability, is increasing. However, from the aspects of low fuel consumption and energy saving, further improvement in viscosity index and low-temperature viscosity characteristics is required.

For example, Patent Literature 1 discloses an α-olefin polymer with a number average molecular weight (Mn) of 500 to 15,000 which contains a constitutional unit derived from α-olefin having 8 to 20 carbon atoms within the range of 50 to 100 mol%, and a synthetic lubricating oil consisting thereof. Patent Literature 1 discloses that the synthetic lubricating oil exhibits a superior viscosity index and low-temperature viscosity characteristics.

For example, Patent Literature 2 discloses an α-olefin polymer with a number average molecular weight (Mn) of 500 to 15,000 which contains a constitutional unit derived from α-olefin having 8 to 20 carbon atoms within the range of 90 to 100 mol%, and a synthetic lubricating oil consisting thereof. Patent Literature 2 discloses that the synthetic lubricating oil exhibits a superior viscosity index and low-temperature viscosity characteristics.

In the field of information and electronics, electronic components compatible with 5G communications, display components compatible with flexibilization, and the like have been developed in recent years. These electronic components use polyolefin-based resins and additives as materials with low dielectric constants, low dielectric loss tangents, superior heat resistance, and strain resistance, in order to suppress low latency due to transmission loss and to enable use in high-temperature environments.

For example, Patent Literature 3 discloses the use of polyolefin-based resins such as polyisobutylene resin, maleic anhydride group-containing propylene-butene copolymer, and the like in a sealing resin composition that suppresses outgassing due to thermal degradation. Patent Literature 3 also discloses the use of a liquid poly-α-olefin having a weight average molecular weight of 500 to 5000 as a plasticizer, from the aspect of adhesiveness.

For example, Patent Literature 4 discloses that an adhesive sheet including an adhesive composition layer containing an isobutylene-based polymer and an ethylene-α-olefin oligomer as a plasticizer is superior in adhesiveness, responsiveness to strain, and recovery property.

In the field of cosmetics, liquid paraffin, ester oil, wax, oil and fat, and higher alcohol are frequently used conventionally to prevent evaporation of water from the skin. Generally, the higher the molecular weight of a base material for cosmetics, the more effective it is in preventing water evaporation. However, when a high-molecular-weight base material for cosmetics is used, stickiness tends to occur. In recent years, consumers demand a sense of use with a luxurious feel, and base materials for cosmetics that can produce a luxury feel are demanded. A luxury feel refers to a feeling of melting on the skin, with a rich texture on application but no sticky feel.

Furthermore, in recent years, from the aspects of improving the texture and moisturizing effect, emulsion cosmetics or oil-based solid cosmetics containing a large amount of wax, oil and fat, and higher alcohols that are solid at room temperature have become popular. However, such cosmetics have the problem that the crystallization of ingredients proceed under low or high temperature conditions and the stability thereof is degraded.

For example, Patent Literature 5 discloses that a cosmetic composition containing at least one branched oligo-α-olefin characterized in that the side chain at the branch point is an ethyl, propyl or longer branched alkyl chain is not sticky and exhibits good smoothness when applied to the skin.

For example, Patent Literature 6 discloses that a water-in-oil cosmetic containing an α-olefin oligomer having a weight average molecular weight of 5000 to 60000 and a viscosity value at 40°C of 1000 cp to 40000 cp, and a polyoxyalkylene-modified organopolysiloxane and/or a long-chain alkyl group-containing polyoxyalkylene-modified organopolysiloxane having an HLB of 2 to 8, has good spreadability on the skin, superior adhesion to the skin, and a high emollient effect.

### [Citation List]

### [Patent Literature]

[Patent Literature 1]
   JP 2005-200446 A
[Patent Literature 2]
   JP 2006-176760 A
[Patent Literature 3]
   JP 2017-27941 A
[Patent Literature 4]
   JP 2021-54980 A
[Patent Literature 5]
   JP 2006-523635 A
[Patent Literature 6]
   JP 2001-72533 A

### [Summary of Invention]

### [Technical Problem]

Conventional polyolefins containing additives used in plasticizers for electronic materials sometimes show precipitation of the aforementioned additives and a decrease in plasticity, after temperature cycles from -20°C to 90°C, assuming use in winter.

Furthermore, although various attempts have been made to improve the sense of use of cosmetics, when conventional polyolefins are used as base materials for cosmetics, the effect of suppressing transdermal water evaporation, the absence of sticky feel, and the effect of suppressing crystallization are sometimes not fully satisfied.

Furthermore, cosmetics containing a conventional polyolefin as a base material for cosmetics and containing large amounts of components that are solid at room temperature (e.g., wax, oil and fat, higher alcohol, etc.) sometimes show progressed crystallization of ingredients and degraded stability thereof under harsh conditions of temperature cycles from -20°C to 50°C assuming winter bathroom temperatures. Furthermore, when attempting to impart a rich texture to the aforementioned cosmetic, a sticky feel may occur or melting feel on the skin may decrease.

Furthermore, conventional polyolefins containing additives used in lubricating oils sometimes show a decrease in lubricity after temperature cycles from -20°C to 90°C, assuming use in winter.

The problem of the present invention is to provide a polyolefin useful as a plasticizer that can suppress precipitation of the additives blended in a plasticizer for electronic materials, even after temperature cycles from -20°C to 90°C assuming use in winter.

Furthermore, the problem of the present invention is to provide a polyolefin useful as a base material for cosmetics superior in the effect of suppressing transdermal water evaporation and the effect of suppressing crystallization, and having less sticky feel.

Furthermore, the problem of the present invention is to provide cosmetics superior in the stability under temperature cycle conditions of from -20°C to 50°C, rich texture and melting feel on the skin, and having less sticky feel, even when they contain large amounts of components that are solid at room temperature (e.g., wax, oil and fat, higher alcohol, etc.).

Furthermore, the problem of the present invention is to provide a polyolefin useful as a lubricant showing superior lubricity even after temperature cycles from -20°C to 90°C assuming use in winter.

### [Solution to Problem]

The present inventors have conducted intensive studies in view of the above-mentioned situation and found that a novel polyolefin having a number average molecular weight in a specific range and a rate of side chains having (n-3) carbon atoms or lower than (n-3) carbon atoms in a specific range can solve the above-mentioned problems. The present invention based on this finding is as follows.

[1] A polyolefin having a number average molecular weight of 250 to 10,000 and obtained from a linear olefin monomer having 8 to 20 carbon atoms, wherein a rate of side chains having (n-3) carbon atoms or lower than (n-3) carbon atoms is 1 to 40%, as calculated from the formula (1): rate of side chains having (n-3) carbon atoms or lower than (n-3) carbon atoms (%) = [Nx/(Nx+Ny)×2n-3]/3×100 ··· [in the formula (1),
   n is an average number of carbon atoms of the aforementioned olefin monomer,
   Nx is an integral value of a signal at 0.75 to 0.95 ppm detected in ¹H-NMR measurement of the aforementioned polyolefin, and
   Ny is an integral value of a signal at 1.00 to 1.45 ppm detected in ¹H-NMR measurement of the aforementioned polyolefin] .
[1a] The polyolefin of the aforementioned [1], wherein the linear olefin monomer having 8 to 20 carbon atoms comprises at least one selected from the group consisting of 1-decene, 1-dodecene and 1-tetradecene, more preferably 1-decene and/or 1-dodecene, further preferably 1-decene.
[1b] The polyolefin of the aforementioned [1] or [1a], wherein the number average molecular weight is 300 to 5,000, more preferably 400 to 2,500, further preferably 400 to 1,800.
[1c] The polyolefin of any one of the aforementioned [1] to [1b], wherein the rate of side chains having (n-3) carbon atoms or lower than (n-3) carbon atoms is 1 to 30%, more preferably 1 to 20%, further preferably 5 to 20%.
[2] A method for producing the polyolefin of any one of the aforementioned [1] to [1c], comprising polymerizing a linear olefin monomer having 8 to 20 carbon atoms in the presence of a Lewis acid catalyst.
[2a] The method of the aforementioned [2], wherein a supply rate of the aforementioned olefin monomer is changed when 35 to 75% by mass, more preferably 40 to 75% by mass, further preferably 50 to 75% by mass, of the aforementioned olefin monomer is supplied with respect to the total supply amount of the aforementioned olefin monomer, and V1/V2 wherein V1 is a supply rate of the aforementioned olefin monomer before the change and V2 is a supply rate of the aforementioned olefin monomer after the change is 0.1 or more and less than 1.0, more preferably 0.1 or more and less than 0.75, further preferably 0.1 or more and less than 0.6, particularly preferably 0.5.
[2b] The method of the aforementioned [2] or [2a], wherein the reaction temperature is increased by 1 to 20°C, more preferably 1 to 15°C, further preferably 1 to 10°C, when 35 to 75% by mass of the aforementioned olefin monomer is supplied with respect to the total supply amount of the aforementioned olefin monomer.
[3] A plasticizer for electronic materials, consisting of the polyolefin of any one of the aforementioned [1] to [1c].
[3a] Use of the polyolefin of any one of the aforementioned [1] to [1c] as a plasticizer for electronic materials.
[4] A base material for cosmetics, consisting of the polyolefin of any one of the aforementioned [1] to [1c].
[4a] Use of the polyolefin of any one of the aforementioned [1] to [1c] as a base material for cosmetics.
[5] A cosmetic comprising the base material for cosmetics of the aforementioned [4] at a content of 1 to 80% by mass with respect to the whole cosmetic.
[6] A lubricant consisting of the polyolefin of any one of the aforementioned [1] to [1c].
[6a] Use of the polyolefin of any one of the aforementioned [1] to [1c] as a lubricant.

### [Advantageous Effects of Invention]

According to the present invention, a polyolefin useful as a plasticizer that can suppress precipitation of the additives blended in a plasticizer for electronic materials, even after temperature cycles from -20°C to 90°C assuming use in winter, can be obtained.

According to the present invention, moreover, a polyolefin useful as a base material for cosmetics superior in the effect of suppressing transdermal water evaporation and the effect of suppressing crystallization, and having less stickiness can be obtained.

According to the present invention, moreover, cosmetics superior in the stability under temperature cycle conditions of from -20°C to 50°C, rich texture and melting feel on the skin, and having less sticky feel, even when they contain large amounts of components that are solid at room temperature (e.g., wax, oil and fat, higher alcohol, etc.) can be obtained.

According to the present invention, moreover, a polyolefin useful as a lubricant showing superior lubricity even after temperature cycles from -20°C to 90°C assuming use in winter can be obtained.

### [Description of Embodiments]

The descriptions in the present specification can be combined with each other unless it is clear that they cannot be combined. In addition, in the present specification, a numerical range defined using "to" includes the numerical values at both ends (upper and lower limits) connected with "to". For example, "2 to 5" means 2 or more and 5 or less.

### (Polyolefin)

The polyolefin of the present invention is a polyolefin obtained from a linear olefin monomer having 8 to 20 carbon atoms. Only one of the aforementioned olefin monomers may be used, or two or more may be used in combination. Examples of the aforementioned olefin monomer include 1-octene, 1-nonene, 1-decene, 2-decene, 3-decene, 4-decene, 5-decene, 1-undecene, 1-dodecene, 1-tridecene, 1-tetradecene, 1-pentadecene, 1-hexadecene, butadiene, and the like. The aforementioned olefin monomer has preferably 8 to 16, more preferably 10 to 15, further preferably 10 to 12, particularly preferably 10, carbon atoms.

The ratio of α-olefin in the aforementioned olefin monomer is not particularly limited, and is preferably 70 to 100% by mass, more preferably 80 to 100% by mass.

The aforementioned olefin monomer preferably includes at least one selected from the group consisting of 1-decene, 1-dodecene and 1-tetradecene, more preferably 1-decene and/or 1-dodecene, further preferably 1-decene.

The number average molecular weight (Mn) of the polyolefin of the present invention is determined from a chromatogram obtained by using a differential refractometer in gel permeation chromatography (GPC) using polystyrene as the standard and tetrahydrofuran as the developing solvent, that is, a graph showing the relationship between refractive index intensity and elution time. The number average molecular weight of the polyolefin of the present invention is 250 to 10,000, preferably 300 to 5,000, more preferably 400 to 2,500, further preferably 400 to 1,800. When the number average molecular weight of polyolefin is less than 250, the effect of polyolefin on suppressing crystallization of additives (hereinafter sometimes referred to as "crystallization-suppressing effect") and the rich texture of the obtained cosmetic are degraded. When the number average molecular weight thereof exceeds 10,000, the handling property of polyolefin becomes poor, and the obtained cosmetic unpreferably shows sticky feel.

One of the features of the polyolefin of the present invention is a rate of side chains having (n-3) carbon atoms or lower than (n-3) carbon atoms of 1 to 40%. As used herein, "n" means an average number of carbon atoms of the aforementioned olefin monomer (i.e., arithmetic mean of the carbon atoms of the aforementioned olefin monomer), and "rate of side chains having (n-3) carbon atoms or lower than (n-3) carbon atoms" means the ratio (%) of the number of side chains having (n-3) carbon atoms or lower than (n-3) carbon atoms with respect to the total number of side chains in the polyolefin (=100 × number of side chains having (n-3) carbon atoms or lower than (n-3) carbon atoms/total number of side chains). For example, when n is 10, the "rate of side chains having (n-3) carbon atoms or lower than (n-3) carbon atoms" means the "rate of side chains having 7 carbon atoms or lower than 7 carbon atoms".

The rate of side chains having (n-3) carbon atoms or lower than (n-3) carbon atoms of polyolefin can be calculated from the formula (1): rate of side chains having (n-3) carbon atoms or lower than (n-3) carbon atoms (%) = [Nx/(Nx+Ny)×2n-3]/3×100 ··· [in the formula (1),
n is an average number of carbon atoms of the aforementioned olefin monomer,
Nx is an integral value of a signal at 0.75 to 0.95 ppm detected in ¹H-NMR measurement of the aforementioned polyolefin, and
Ny is an integral value of a signal at 1.00 to 1.45 ppm detected in ¹H-NMR measurement of the aforementioned polyolefin].

The rate of side chains having (n-3) carbon atoms or lower than (n-3) carbon atoms of polyolefins can be calculated, for example, by the following procedure. Specifically, a 600 Hz NMR measurement device (JNM-ECA600) manufactured by JEOL Ltd. was used, and a mixed solution of analysis sample: deuterated chloroform = 1:45 (mass ratio) was placed in an NMR sample tube with an inner diameter of 5 mm to prepare a measurement sample, and ¹H-NMR measurement of this measurement sample was performed under the conditions of relaxation delay time: 5 seconds and induced free decay collection time: 1.8 seconds. From the obtained ¹H-NMR spectrum, the integral value (Nx) of the signal from 0.75 to 0.95 ppm and the integral value (Ny) of the signal from 1.00 to 1.45 ppm are calculated, with the signal of trimethylsilane (TMS) set at 0 ppm. Note that Nx is the integral value of the signal of CH₃, and Ny is the integral value of the signals of CH₂ and CH. When the polyolefin has a side chain having (n-3) carbon atoms or lower than (n-3) carbon atoms, the number of protons derived from the terminal methyl group in the structural unit of the polyolefin increases by 3 compared to when the polyolefin does not have such a side chain. Therefore, the rate of side chains having (n-3) carbon atoms or lower than (n-3) carbon atoms can be calculated from the above formula (1) using the obtained Nx and Ny and the average number of carbon atoms (n) of the linear olefin monomer.

When the rate of side chains having (n-3) carbon atoms or lower than (n-3) carbon atoms is less than 1%, the crystallization-suppressing effect decreases. When the rate of side chains having (n-3) carbon atoms or lower than (n-3) carbon atoms exceeds 40%, the crystallization-suppressing effect decreases, and the melting feel on the skin of the obtained cosmetic further decreases. The rate of side chains having (n-3) carbon atoms or lower than (n-3) carbon atoms is preferably 1 to 30%, more preferably 1 to 20%, further preferably 5 to 20%.

### (Production method of polyolefin)

The polyolefin of the present invention is preferably obtained by a production method including polymerizing a linear olefin monomer having 8 to 20 carbon atoms in the presence of a Lewis acid catalyst. The polymerization may be performed using an organic solvent or without a solvent. Specifically, the polyolefin of the present invention can be obtained by a production method including charging an initiator and, if necessary, an organic solvent into a reaction vessel, then supplying a Lewis acid catalyst, and adding the aforementioned olefin monomer continuously or intermittently while stirring the mixture of the organic solvent, the initiator, and the Lewis acid catalyst, preferably in an inert gas atmosphere, to perform addition polymerization. The olefin monomer may be added under pressure or atmospheric pressure.

A supply rate of the olefin monomer is more preferably changed when 35 to 75% by mass, more preferably 40 to 75% by mass, further preferably 50 to 75% by mass, of the olefin monomer is supplied with respect to the total supply amount of the olefin monomer. V1/V2 wherein V1 is a supply rate (supply amount per unit time) of the olefin monomer before the change and V2 is a supply rate of the olefin monomer after the change is 0.1 or more and less than 1.0, more preferably 0.1 or more and less than 0.75, further preferably 0.1 or more and less than 0.6, particularly preferably 0.5.

The reaction temperature during polymerization of polyolefins is, for example, -10°C to 100°C, preferably -10°C to 50°C, further preferably -10°C to 30°C.

The reaction temperature is desirably increased by preferably 1 to 20°C, more preferably 1 to 15°C, further preferably 1 to 10°C, when 35 to 75% by mass of olefin monomer is supplied to the total supply amount of olefin monomer.

The reaction pressure during polymerization of polyolefin is not particularly limited and it is, for example, 0 to 2.0 MPa, preferably 0.1 to 1.0 MPa.

Examples of the Lewis acid catalyst to be used in polymerization of olefin monomers include metal halides or organometallic halides such as SnCl₄, TiCl₄, TiCl₃, TiBr₄, TiBr₃, VCl₅, FeCl₃, ZrCl₃, ZrCl₄, ZnBr₂, BF₃, BCl₃, AlCl₃, and the like. From the aspect of reactivity, an aluminum compound is preferred as the Lewis acid catalyst, and AlCl₃ is more preferred. The "metal" in the aforementioned "metal halide or organometallic halide" also includes "metalloid".

The amount of Lewis acid catalyst to olefin monomer (i.e., 100 × amount of Lewis acid catalyst used (mol)/amount of olefin monomer used (mol)) is, for example, 0.01 to 10 mol%, preferably 0.01 to 5 mol%, more preferably 0.1 to 5 mol%.

Examples of the initiator to be used in polymerization of olefin monomers include water, alcohols, and the like, and water is preferred. The amount of initiator to Lewis acid catalyst (i.e., 100 × amount of initiator used (mol)/amount of Lewis acid catalyst used (mol)) is, for example, 0.01 to 200 mol%, preferably 1 to 200 mol%, more preferably 10 to 150 mol%, further preferably 10 to 100 mol%.

Examples of the organic solvent to be used in polymerization of olefin monomers include hydrocarbons such as hexane, heptane, octane, decane, liquid paraffin, and the like, aromatic hydrocarbons such as toluene, xylene, and the like, dimethyl sulfoxide, amides such as N,N-dimethylformamide, dimethylacetamide, acetoanilide, and the like, ketones such as acetone, methyl ethyl ketone, methyl isobutyl ketone, cyclohexanone, and the like, halogenated hydrocarbons such as dichloroethane, dichloromethane, and the like, acetates such as methyl acetate, ethyl acetate, propyl acetate, butyl acetate, methyl butyrate, and the like. These may be used alone or in combination with two or more kinds thereof. From the aspect of reactivity, the dielectric constant of the organic solvent at 25°C is preferably 1 to 50, more preferably 1 to 30, further preferably 1 to 10, particularly preferably 1 to 5. The dielectric constant of the organic solvent at 25°C can be measured using a dielectric constant meter for liquids Model871 (manufactured by Nihon Rufuto Co., Ltd.).

The amount of organic solvent to olefin monomer (i.e., 100 × amount of organic solvent used (g)/amount of olefin monomer used (g)) is preferably 50 to 10, 000% by mass, more preferably 50 to 500% by mass, further preferably 50 to 200% by mass.

The Lewis acid catalyst must be deactivated by adding a quencher to the reaction solution removed from the reactor. As the quencher, for example, water, alcohols, ethers, amines, acetonitrile, ammonia, a sodium hydroxide aqueous solution, a potassium hydroxide aqueous solution, or the like can be used. Of these, water or a sodium hydroxide aqueous solution preferably can be used.

### (Lubricant)

The polyolefin of the present invention is preferably used as a lubricant. Therefore, the present invention also provides a lubricant consisting of the polyolefin of the present invention. The applicable use of the lubricant consisting of the polyolefin of the present invention is not particularly limited, and the lubricant can be preferably used as, for example, gear oil, engine oil, industrial lubricating oil, hydraulic oil, or grease.

The lubricant consisting of the polyolefin of the present invention may be used in combination with other base oil and additives for lubricants. Examples of other base oil include mineral oil, synthetic oil, and the like. Examples of the mineral oil include hydrogenated refined oil, paraffin-based mineral oil, and the like. Examples of the synthetic oil include oil synthesized by the Fischer-Tropsch reaction using gass such as methane as a starting material, poly-α-olefin oligomer, polybutene, alkylbenzene, monoester, polyol ester, polyglycol ester, polyethylene propylene, hindered ester, dibasic acid ester, and the like.

Examples of the additive for lubricants include antioxidant, oiliness improver, anti-wear agent, extreme pressure agent, metal deactivator, antifoaming agent, rust inhibitor, pour point depressant, viscosity index improver, hydrolysis inhibitor, and the like. Examples of the antioxidant include zinc dialkyldithiophosphate, organosulfur compound, hindered phenol, aromatic amine, and the like. Examples of the oiliness improver include higher fatty acid (e.g., fatty acid having 13 to 21 carbon atoms), fatty acid ester, higher alcohol, alkylamine, and the like. Examples of the anti-wear agent include phosphate ester and the like. Examples of the extreme pressure agent include organosulfur, phosphorus compound, and organohalogen compound. Examples of the metal deactivator include benzotriazole, N,N'-disalicylidene-1,2-diaminopropane, and the like. Examples of the antifoaming agent include polymethylsiloxane, organic fluorine compound, metal soap, fatty acid ester, phosphate ester, higher alcohol, polyalkylene glycol compound, and the like. The additive for lubricants is preferably an oiliness improver, more preferably higher fatty acid, further preferably fatty acid having 13 to 21 carbon atoms.

When the lubricant of the present invention is used as an engine oil, precipitation of the additive for lubricants can be suppressed. Therefore, good lubrication performance can be exhibited even after temperature cycles from -20°C to 90°C. That is, a lubricant composition containing the additives for lubricants and the lubricant of the present invention (namely, the polyolefin of the present invention) can exhibit good lubrication performance.

As additives for lubricants that can be used in a lubricant composition, the aforementioned can be mentioned. The lubricant composition may also contain other base oils mentioned above. The content of the lubricant of the present invention (i.e., the polyolefin of the present invention) in the lubricant composition is preferably 40 to 99.9% by mass, more preferably 70 to 99.9% by mass, further preferably 80 to 99.8% by mas, of the whole lubricant composition.

In the lubricant composition, the additive for lubricants and the lubricant of the present invention (i.e., the polyolefin of the present invention), and any other base oil may be used alone or in combination with two or more kinds thereof.

### (Plasticizer for electronic materials)

The polyolefin of the present invention is preferably used as a plasticizer for electronic materials. Therefore, the present invention also provides a plasticizer for electronic materials consisting of the polyolefin of the present invention. The applicable use of the plasticizer for electronic materials of the present invention is not particularly limited, but the plasticizer for electronic materials of the present invention can be suitably used, for example, as a plasticizer for sealing materials for flexible electronic devices, semiconductors, and the like. The plasticizer for electronic materials of the present invention may be used in combination with other components (e.g., other plasticizers).

Particularly when the plasticizer for electronic materials of the present invention is used as the aforementioned plasticizer for sealing materials, precipitation of the additive used with the aforementioned plasticizer can be suppressed. Therefore, good plasticity performance can be exhibited even after temperature cycles from -20°C to 90°C. That is, an electronic material composition containing electronic materials and the polyolefin of the present invention can exhibit good plasticity.

The content of the plasticizer for electronic materials of the present invention (i.e., the polyolefin of the present invention) in the electronic material composition is preferably 1 to 50 parts by mass, more preferably 5 to 25 parts by mass with respect to 100 parts by mass of the electronic material. The electronic material composition may contain additives other than the electronic material or the plasticizer for electronic materials of the present invention.

As the electronic material, a thermoplastic resin is preferred, and a polyolefin obtained from an olefin monomer having 2 to 7 carbon atoms is preferred.

In the electronic material composition, the electronic material and the plasticizer for electronic materials of the present invention (i.e., the polyolefin of the present invention), and any additives may be used alone or in combination with two or more kinds thereof.

### (Base material for cosmetics)

The polyolefin of the present invention is preferably used as a base material for cosmetics. Therefore, the present invention also provides a base material for cosmetics consisting of the polyolefin of the present invention. The applicable use of the base material for cosmetics of the present invention is not particularly limited. The base material for cosmetics of the present invention can be suitably used as a base material for cosmetics that is superior in the effect of suppressing transdermal water evaporation and the effect of suppressing crystallization, and has little sticky feel. The base material for cosmetics of the present invention may be used in combination with other components (e.g., other base materials, cosmetic additives).

Examples of the cosmetic containing the base material for cosmetics of the present invention include creams (e.g., face cream, body cream, sunscreen cream, moisturizing cream, hand cream, body cream, foot cream, massage cream), milky lotion, foundations (e.g., liquid foundation), oil serum, massage oil, hair oil, cleansing oil, sunscreen, point makeup remover, treatment, mascara, lipstick, and the like.

Particularly, cosmetics containing the base material for cosmetics of the present invention can exhibit good stability even after temperature cycles from -20°C to 90°C, show rich texture on application, cause no sticky feel, and can offer a feeling of melting on the skin.

### (Cosmetics)

The present invention also provides a cosmetic containing the base material for cosmetics of the present invention in a content of 1 to 80% by mass with respect to the whole cosmetic. That is, the present invention also provides a cosmetic containing 1 to 80% by mass of the base material for cosmetics of the present invention (i.e., the polyolefin of the present invention) and 20 to 99% by mass of other cosmetic components, with respect to the whole cosmetic. In the present specification, "other cosmetic components" means cosmetic components that are different from the base material for cosmetics of the present invention. When the content of the base material for cosmetics of the present invention is less than 1% by mass, the effect cannot be fully exhibited, and when the content of the base material for cosmetics of the present invention is more than 80% by mass, the stability of the cosmetic may be deteriorated. The content of the base material for cosmetics of the present invention with respect to the whole cosmetic is preferably 1 to 50% by mass, more preferably 1 to 30% by mass.

Examples of the cosmetic are as mentioned above. Examples of other cosmetic component include solvent, oils and fats, higher fatty acids (e.g., fatty acid having 13 to 21 carbon atoms), higher alcohol, silicone, esters, surfactant, humectant, thickener, antioxidant, stabilizer, antiseptic, pearlizing agent, pH adjuster, ultraviolet absorber, hydrotope agent, pigment, dye, bactericide, anti-inflammatory agent, astringent, algefacient, flavor, plant extract, and the like. Examples of the solvent include water, ethanol, 1,3-butyleneglycol, dipropylene glycol, propylene glycol, glycerin, and the like.

In the cosmetic of the present invention, other cosmetic component and the base material for cosmetics of the present invention (i.e., the polyolefin of the present invention) may be used alone or in combination with two or more kinds thereof.

### [Example]

The present invention is described in detail below with Examples and Comparative Examples.

### <Example 1: Synthesis of polyolefin 1>

A four-neck flask with an internal volume of 3 L was charged with 750 ml of heptane and 1.8 g of deionized water, and 35 mmol of aluminum chloride was supplied per 1 mol of 1-dodecene at 40°C. Thereafter, 1-dodecene was fed at a flow rate of 0.4 L/h while maintaining the reactor at 40°C up to 375 ml, and from 375 ml onwards, 1-dodecene was fed at a flow rate of 0.4 L/h while maintaining the reactor at 60°C to perform polymerization reaction. Subsequently, 750 ml of a 20% by mass aqueous sodium hydroxide solution was added to the reaction solution at 80°C to deactivate the Lewis acid catalyst and perform neutralization washing twice, and the organic layer was further washed six times with 750 ml of deionized water, and desolvation and dehydration were performed at 100°C and under reduced pressure of 6.7 kPa or less, followed by adsorption treatment at 80°C with an adsorbent ("KW-700" and "KW-1000", Kyowa Chemical Industry Co., Ltd.) to recover polyolefin 1. The rate of side chains having (n-3) carbon atoms or lower than (n-3) carbon atoms in polyolefin 1 calculated by ¹H-NMR and GPC was 28%.

### <Example 2: Synthesis of polyolefin 2>

A four-neck flask with an internal volume of 3 L was charged with 750 ml of heptane and 0.9 g of deionized water, and 17.5 mmol of aluminum chloride was supplied per 1 mol of 1-decene at 10°C. Thereafter, 1-dodecene was fed at a flow rate of 0.3 L/h while maintaining the reactor at 10°C up to 375 ml, and from 375 ml onwards, 1-dodecene was fed at a flow rate of 0.6 L/h while maintaining the reactor at 20°C to perform polymerization reaction. Subsequently, 750 ml of a 20% by mass aqueous sodium hydroxide solution was added to the reaction solution at 80°C to deactivate the Lewis acid catalyst and perform neutralization washing twice, and the organic layer was further washed six times with 750 ml of deionized water, and desolvation and dehydration were performed at 100°C and under reduced pressure of 6.7 kPa or less, followed by adsorption treatment at 80°C with an adsorbent ("KW-700" and "KW-1000", Kyowa Chemical Industry Co., Ltd.) to recover polyolefin 2. The rate of side chains having (n-3) carbon atoms or lower than (n-3) carbon atoms in polyolefin 2 calculated by ¹H-NMR and GPC was 11%.

### <Example 3: Synthesis of polyolefin 3>

A four-neck flask with an internal volume of 3 L was charged with 750 ml of hexane and 1.8 g of deionized water, and 35 mmol of aluminum chloride was supplied per 1 mol of an olefin monomer mixture (1-decene (45% by mass) and 1-dodecene (55% by mass)) at 50°C. Thereafter, the olefin monomer mixture was fed at a flow rate of 0.2 L/h while maintaining the reactor at 50°C up to 300 ml, and from 300 ml onwards, the olefin monomer mixture was fed at a flow rate of 1 L/h while maintaining the reactor at 55°C to perform polymerization reaction. Subsequently, 750 ml of a 20% by mass aqueous sodium hydroxide solution was added to the reaction solution at 80°C to deactivate the Lewis acid catalyst and perform neutralization washing twice, and the organic layer was further washed six times with 750 ml of deionized water, and desolvation and dehydration were performed at 100°C and under reduced pressure of 6.7 kPa or less, followed by adsorption treatment at 80°C with an adsorbent ("KW-700" and "KW-1000", Kyowa Chemical Industry Co., Ltd.) to recover polyolefin 3. The rate of side chains having (n-3) carbon atoms or lower than (n-3) carbon atoms in polyolefin 3 calculated by ¹H-NMR and GPC was 20%.

### <Example 4: Synthesis of polyolefin 4>

A four-neck flask with an internal volume of 3 L was charged with 750 ml of heptane and 4.5 g of deionized water, and 35 mmol of aluminum chloride was supplied per 1 mol of 1-decene at 40°C. Thereafter, 1-dodecene was fed at a flow rate of 0.3 L/h while maintaining the reactor at 40°C up to 150 ml, and from 150 ml onwards, 1-dodecene was fed at a flow rate of 0.4 L/h while maintaining the reactor at 40°C to perform polymerization reaction. Subsequently, 750 ml of a 20% by mass aqueous sodium hydroxide solution was added to the reaction solution at 80°C to deactivate the Lewis acid catalyst and perform neutralization washing twice, and the organic layer was further washed six times with 750 ml of deionized water, and desolvation and dehydration were performed at 100°C and under reduced pressure of 6.7 kPa or less, followed by adsorption treatment at 80°C with an adsorbent ("KW-700" and "KW-1000", Kyowa Chemical Industry Co., Ltd.) to recover polyolefin 4. The rate of side chains having (n-3) carbon atoms or lower than (n-3) carbon atoms in polyolefin 4 calculated by ¹H-NMR and GPC was 25%.

### <Example 5: Synthesis of polyolefin 5>

1-Tetradecene was fed into a stainless steel 5-liter (inner volume 4,890 ml) pressure-resistant reactor at a flow rate of 0.4 L/h up to 750 ml while maintaining the reactor at 80°C, and 74 mmol of boron trifluoride (BF₃) was fed per 1 mol of 1-tetradecene at 40°C. Diethyl ether and ethanol were charged such that the molar ratios to boron trifluoride were 1.00 and 0.03, respectively, and a polymerization reaction was performed while maintaining the temperature at 40°C. Subsequently, 750 ml of a 20% aqueous sodium hydroxide solution was added to the reaction solution at 80°C to deactivate the Lewis acid catalyst and perform neutralization washing twice, and the organic layer was further washed six times with 750 ml of deionized water, and desolvation and dehydration were performed at 100°C and under reduced pressure of 6.7 kPa or less, followed by adsorption treatment at 80°C with an adsorbent ("KW-700" and "KW-1000", Kyowa Chemical Industry Co., Ltd.) to recover polyolefin 5. The rate of side chains having (n-3) carbon atoms or lower than (n-3) carbon atoms in polyolefin 5 calculated by ¹H-NMR and GPC was 38%.

### <Comparative Example 1: Synthesis of polyolefin C1>

A four-neck flask with an internal volume of 5 L was charged with 750 ml of dichloroethane and 1.8 g of deionized water, and 74 mmol of aluminum chloride was supplied per 1 mol of 1-decene at 15°C. Thereafter, 7500 ml of 1-decene was fed at a flow rate of 0.4 L/h and polymerization reaction was performed at a reactor temperature of 50°C. Subsequently, 750 ml of a 20% by mass aqueous sodium hydroxide solution was added to the reaction solution at 80°C to deactivate the Lewis acid catalyst and perform neutralization washing twice, and the organic layer was further washed six times with 750 ml of deionized water, and desolvation and dehydration were performed at 100°C and under reduced pressure of 6.7 kPa or less, followed by adsorption treatment at 80°C with an adsorbent ("KW-700" and "KW-1000", Kyowa Chemical Industry Co., Ltd.) to recover polyolefin C1. The rate of side chains having (n-3) carbon atoms or lower than (n-3) carbon atoms in polyolefin C1 calculated by ¹H-NMR and GPC was 31%.

### <Comparative Example 2: Synthesis of polyolefin C2>

A four-neck flask with an internal volume of 5 L was charged with 325 ml of heptane and 1.8 g of deionized water, and 74 mmol of aluminum chloride was supplied per 1 mol of 1-decene at 15°C. Thereafter, 325 ml of 1-decene was fed at a flow rate of 0.4 L/h and polymerization reaction was performed at a reactor temperature of 130°C. Subsequently, 325 ml of a 20% by mass aqueous sodium hydroxide solution was added to the reaction solution at 80°C to deactivate the Lewis acid catalyst and perform neutralization washing twice, and the organic layer was further washed six times with 325 ml of deionized water, and desolvation and dehydration were performed at 100°C and under reduced pressure of 6.7 kPa or less, followed by adsorption treatment at 80°C with an adsorbent ("KW-700" and "KW-1000", Kyowa Chemical Industry Co., Ltd.) to recover polyolefin C2. The rate of side chains having (n-3) carbon atoms or lower than (n-3) carbon atoms in polyolefin C2 calculated by ¹H-NMR and GPC was 52%.

The number average molecular weight of each polyolefin obtained in Examples 1 to 5 and Comparative Examples 1 and 2 was measured by gel permeation chromatography (GPC).

The olefin monomers used in Examples 1 to 5 and Comparative Examples 1 and 2, as well as the number average molecular weight and rate of side chains having (n-3) carbon atoms or lower than (n-3) carbon atoms of the obtained polyolefins are shown in Table 1.

**[Table 1]**

| | Example 1 polyolefin 1 | Example 2 polyolefin 2 | Example 3 polyolefin 3 | Example 4 polyolefin 4 | Example 5 polyolefin 5 | Comparative Example 1 polyolefin C1 | Comparative Example 2 polyolefin C2 |
|---|---|---|---|---|---|---|---|
| olefin monomer | 1-dodecene | 1-decene | 1-decene (45% by mass) 1-dodecene (55% by mass) | 1-decene | 1-tetradecene | 1-decene | 1-dodecene |
| number average molecular weight | 1978 | 1550 | 1847 | 444 | 678 | 14869 | 1562 |
| rate of side chains having (n-3) carbon atoms or lower than (n-3) carbon atoms (%) | 28 | 11 | 20 | 25 | 38 | 31 | 52 |

### <Evaluation of characteristic as plasticizer for electronic materials>

The characteristic (plasticity) of the polyolefins obtained in Examples 1 to 5 and Comparative Examples 1 and 2 as plasticizers for electronic materials were evaluated by the following method. The results thereof are shown in Table 2.

### Evaluation of plasticity

A mixture of 10 g of a toluene solution of polybutylene (polybutylene concentration: 30% by mass), 0.3 g of polyolefin (plasticizer for electronic materials) (amount relative to polybutylene: 10% by mass), and 0.015 g of oleic acid (amount relative to polybutylene: 0.5% by mass) was prepared. The "amount of each component relative to polybutylene" was calculated from the formula "100 × amount of each component used (g)/amount of polybutylene used (g)".

The prepared mixture was applied to a polyethylene terephthalate (PET) film at a speed of 50 mm/s using an automatic coating device, dried at 60°C for 5 min, and then left standing overnight at ordinary temperature to produce a sheet with a thickness of about 30 µm. The prepared sheet was stored in a -20°C storage cabinet for 12 hr, and then stored in a 90°C storage cabinet for 12 hr. After repeating this temperature cycle (1 cycle: 1 day) for 30 cycles (30 days), and the sheet was then cut out to prepare dumbbell-shaped test pieces (JIS K6251-1). The elongation percentage of the dumbbell-shaped test piece was measured by a tensile test using a tensile tester. The obtained elongation percentage was used to evaluate the plasticity of the polyolefin according to the following criteria. Polyolefins evaluated as "O" or "⊙" were assessed to be plasticizers superior in plasticity.

### (Criteria)

⊙: elongation percentage is 120% or more
O: elongation percentage is 110% or more and less than 120%
Δ: elongation percentage is less than 110%

**[Table 2]**

| | Example 1 polyolefin 1 | Example 2 polyolefin 2 | Example 3 polyolefin 3 | Example 4 polyolefin 4 | Example 5 polyolefin 5 | Comparative Example 1 polyolefin C1 | Comparative Example 2 polyolefin C2 |
|---|---|---|---|---|---|---|---|
| plasticity | ○ | ⊙ | ○ | ○ | ○ | Δ | Δ |

As shown in Table 2, polyolefins 1 to 5 obtained in Examples 1 to 5 were superior in plasticity even after a temperature cycle from -20°C to 90°C, compared with the polyolefins C1 and C2 obtained in Comparative Examples 1 and 2.

### <Evaluation of characteristics as base material for cosmetics>

The characteristics of the polyolefins obtained in Examples 1 to 5 and Comparative Examples 1 and 2 as base materials for cosmetics (transdermal water evaporation-suppressing effect, sticky feel, and crystallization-suppressing effect) were evaluated by the following methods. The results thereof are shown in Table 3.

### Evaluation of transdermal water evaporation-suppressing effect

Ten women (age 22 to 40) were used as panelists, the transdermal water evaporation amount before and after application of polyolefin (base material for cosmetics) to the inner forearm was measured using Evaporimeter EP1 (manufactured by YAYOI), and the change ratio of the transdermal water evaporation amount was calculated by the following formula: transdermal water evaporation amount change ratio (%) = [(transdermal water evaporation amount after application of polyolefin - transdermal water evaporation amount before application of polyolefin)/transdermal water evaporation amount before application of polyolefin] × 100

In order to stabilize the body temperature, the panelists waited in a temperature- and humidity-controlled room at a room temperature of 25°C and a humidity of 40% for 30 min or longer, after which the transdermal water evaporation amount was measured in the temperature- and humidity-controlled room.

The average value of change ratio in transdermal water evaporation amount for all panelists was calculated for each polyolefin, and the transdermal water evaporation-suppressing effect was evaluated according to the following criteria. The smaller the aforementioned average value, the more superior the transdermal water evaporation-suppressing effect is.

### (Criteria)

⊙: the aforementioned average value is less than -15%
O: the aforementioned average value is -15% or more and less than -5%
Δ: the aforementioned average value is -5% or more and less than 0%
×: the aforementioned average value is 0% or more

### Evaluation of sticky feel

Twenty women (age 22 to 40) were used as panelists, the obtained polyolefin (base material for cosmetics, about 1 g) was applied to and blended into their skin, and the sticky feel was rated using the following scores and criteria. Polyolefins rated as "O" or "⊙" were assessed to be base materials for cosmetics free of a sticky feel.

### (Scores)

2 points: no sticky feel when applying to and blending into skin
1 point: slight sticky feel when applying to and blending into skin
0 point: sticky feel when applying to and blending into skin

### (Criteria)

⊙: total score is 35 points or more
O: total score is 30 points or more and 34 points or less
Δ: total score is 20 points or more and 29 points or less
×: total score is 19 points or less

### Evaluation of crystallization-suppressing effect

0.1 g of palmitic acid was added to 50 g of each polyolefin, and after thorough mixing and stirring, the mixture was placed in a glass container, sealed, and stored in a 50°C storage cabinet for 12 hr, and then stored in a -20°C storage cabinet for 12 hr. After repeating this temperature cycle (1 cycle: 1 day) for 30 cycles (30 days), the crystallization-suppressing effect was evaluated according to the following criteria.

### (Criteria)

⊙: crystallization of the aforementioned mixture is suppressed at -20°C, and the aforementioned mixture has flowability
O: crystallization of the aforementioned mixture is slightly suppressed at -20°C, and the aforementioned mixture has some flowability
×: the aforementioned mixture is crystallized at -20°C, and the aforementioned mixture has no flowability at all

**[Table 3]**

| | Example 1 polyolefin 1 | Example 2 polyolefin 2 | Example 3 polyolefin 3 | Example 4 polyolefin 4 | Example 5 polyolefin 5 | Comparative Example 1 polyolefin C1 | Comparative Example 2 polyolefin C2 |
|---|---|---|---|---|---|---|---|
| transdermal water evaporation-suppressing effect | ○ | ⊙ | ○ | ○ | ○ | ⊙ | Δ |
| sticky feel | ○ | ⊙ | ○ | ○ | ○ | × | Δ |
| crystallization-suppressing effect | ○ | ⊙ | ○ | ○ | ○ | × | × |

As shown in Table 3, polyolefins 1 to 5 were superior in the transdermal water evaporation-suppressing effect and the crystallization-suppressing effect, and were free of a sticky feel.

On the other hand, polyolefin C1 or C2 was inferior in the transdermal water evaporation-suppressing effect, the absence of sticky feel, and the crystallization-suppressing effect.

### <Evaluation of characteristic as lubricant>

The characteristic (lubricity) of the polyolefins obtained in Examples 1 to 5 and Comparative Examples 1 and 2 as lubricants were evaluated by the following method. The results thereof are shown in Table 4.

### Preparation of lubricant composition

0.1 g of palmitic acid (additive for lubricants) was added to 50 g of each polyolefin (lubricant), and the mixture was thoroughly mixed and stirred to completely dissolve the palmitic acid, whereby a lubricant composition was prepared.

### Evaluation of lubricity before cycle test

Each lubricant composition was placed on a metal disk (SUJ-2), and the static friction coefficient and dynamic friction coefficient before the cycle test were measured using a Bowden tester under the following conditions, the average values of each were calculated, and the lubricity was evaluated according to the following criteria. The lower the aforementioned average value, the more superior the lubricity is.

### (Conditions)

contactor (steel ball, diameter 3 mm)
load: 500 g
temperature: 25°C
rate: 2.5 mm/s
measurement distance: 10 mm
number of tests: 100 times

### (Criteria)

⊙: average static friction coefficient or dynamic friction coefficient is less than 0.100
O: average static friction coefficient or dynamic friction coefficient is 0.100 or more and less than 0.150
Δ: average static friction coefficient or dynamic friction coefficient is 0.150 or more and less than 0.200
×: average static friction coefficient or dynamic friction coefficient is 0.200 or more

### Evaluation of crystallization-suppressing effect

50 g of each lubricant composition was placed in a glass container, sealed, and stored in a 90°C storage cabinet for 12 hr, and then stored in a -20°C storage cabinet for 12 hr. After repeating this temperature cycle (1 cycle: 1 day) for 30 cycles (30 days), the crystallization-suppressing effect was evaluated according to the following criteria.

### (Criteria)

⊙: crystallization of the aforementioned lubricant composition is suppressed at -20°C, and the aforementioned lubricant composition has flowability
O: crystallization of the aforementioned lubricant composition is slightly suppressed at -20°C, and the aforementioned lubricant composition has some flowability
×: the aforementioned lubricant composition is crystallized at -20°C, and the aforementioned lubricant composition has no flowability at all

### Evaluation of lubricity after cycle test

After the cycle test, the lubricant composition was left standing until its temperature reached 25°C. The static friction coefficient and dynamic friction coefficient after the cycle test were measured in the same manner as in the evaluation of lubricity before cycle test, the average values of each were calculated, and the lubricity was evaluated according to the aforementioned criteria. The lower the aforementioned average value, the more superior the lubricity is.

**Table 4]**

| | | Example 1 polyolefin 1 | Example 2 polyolefin 2 | Example 3 polyolefin 3 | Example 4 polyolefin 4 | Example 5 polyolefin 5 | Comparative Example 1 polyolefin C1 | Comparative Example 2 polyolefin C2 |
|---|---|---|---|---|---|---|---|---|
| before cycle test | static friction coefficient | ○ | ⊙ | ○ | ○ | ○ | × | × |
| | | 0.111 | 0.088 | 0.113 | 0.116 | 0.120 | 0.225 | 0.248 |
| | dynamic friction coefficient | ⊙ | ⊙ | ○ | ○ | ○ | × | × |
| | | 0.092 | 0.071 | 0.108 | 0.112 | 0.117 | 0.216 | 0.239 |
| after cycle test | static friction coefficient | ○ | ⊙ | ○ | ○ | ○ | × | × |
| | | 0.110 | 0.087 | 0.115 | 0.115 | 0.123 | 0.262 | 0.286 |
| | dynamic friction coefficient | ⊙ | ⊙ | ○ | ○ | ○ | × | × |
| | | 0.093 | 0.070 | 0.110 | 0.114 | 0.116 | 0.249 | 0.278 |
| | crystallization-suppressing effect | ○ | ⊙ | ○ | ○ | ○ | × | × |

As shown in Table 4, polyolefins 1 to 5 obtained in Examples 1 to 5 were superior in lubricity and the crystallization-suppressing effect as compared to polyolefins C1 and C2 obtained in Comparative Examples 1 and 2.

### <Examples 6 to 12, Comparative Examples 3 and 4: Production of cosmetics>

Using the amounts shown in Table 5, polyolefin (base material for cosmetics) obtained Examples 1 to 5, Comparative Example 1 or 2 and common additive components (wax, etc. that are solid at room temperature) were mixed to produce cosmetics. The obtained cosmetics were evaluated according to the following method. The results thereof are shown in Table 5. In addition, the compositions of common additive components are shown in Table 6.

### Evaluation of temperature cycle stability

50 g of the obtained cosmetic was placed in a glass container, sealed, and stored in a 50°C storage cabinet for 12 hr, and then stored in a -20°C storage cabinet for 12 hr. This temperature cycle (1 cycle: 1 day) was repeated, and the cosmetic was evaluated according to the following criteria.

### (Criteria)

⊙: appearance and property of the cosmetic did not change markedly after 28 cycles
O: appearance and property of the cosmetic changed slightly after 28 cycles, but did not change markedly after 14 cycles
Δ: appearance and property of the cosmetic changed slightly after 14 cycles, but did not change markedly after 7 cycles
×: appearance and property of the cosmetic changed markedly significantly after 7 cycles

### Evaluation of rich texture on application

Twenty women (age 22 to 40) were used as panelists, the obtained cosmetic (about 1 g) was applied to their skin, and the rich texture was rated using the following scores and criteria. Cosmetics rated as "O" or "⊙" were assessed to be cosmetic with a rich texture on application.

### (Scores)

2 points: rich texture when applying to skin
1 point: slight rich texture when applying to skin
0 point: no rich texture when applying to skin

### (Criteria)

⊙: total score is 35 points or more
O: total score is 30 points or more and 34 points or less
Δ: total score is 20 points or more and 29 points or less
×: total score is 19 points or less

### Evaluation of melting feel on the skin

Twenty women (age 22 to 40) were used as panelists, the obtained cosmetic (about 1 g) was applied to and blended into their skin, and the melting feel on the skin was rated using the following scores and criteria. Cosmetics rated as "○" or "⊙" were assessed to be cosmetic with a melting feel on the skin.

### (Scores)

2 points: melting feel on skin when applying to and blending into skin
1 point: slight melting feel on skin when applying to and blending into skin
0 point: no melting feel on skin when applying to and blending into skin

### (Criteria)

⊙: total score is 35 points or more
O: total score is 30 points or more and 34 points or less
Δ: total score is 20 points or more and 29 points or less
×: total score is 19 points or less

### Evaluation of sticky feel

Twenty women (age 22 to 40) were used as panelists, and after applying and blending the obtained cosmetic (about 1 g) into their skin, the sticky feel was rated using the following scores and criteria. Cosmetics rated as "O" or "○" were assessed to be cosmetic free of a sticky feel.

### (Scores)

2 points: no sticky feel after applying to and blending into skin
1 point: slight sticky feel after applying to and blending into skin
0 point: sticky feel after applying to and blending into skin

### (Criteria)

⊙: total score is 35 points or more
O: total score is 30 points or more and 34 points or less
Δ: total score is 20 points or more and 29 points or less
×: total score is 19 points or less

**[Table 5]**

| | | Example 6 | Example 7 | Example 8 | Example 9 | Example 10 | Example 11 | Example 12 | Comparative Example 3 | Comparative Example 4 |
|---|---|---|---|---|---|---|---|---|---|---|
| base material for cosmetics (% by mass) | polyolefin 1 | 20 | | | | | | | | |
| | polyolefin 2 | | 5 | 10 | 40 | | | | | |
| | polyolefin 3 | | | | | 10 | | | | |
| | polyolefin 4 | | | | | | 5 | | | |
| | polyolefin 5 | | | | | | | 10 | | |
| | polyolefin C1 | | | | | | | | 10 | |
| | polyolefin C2 | | | | | | | | | 10 |
| common additive component (% by mass) | | 17.6 | | | | | | | | |
| Water | | rest | | | | | | | | |
| evaluation | temperature cycle stability | O (32) | ⊙ (38) | ⊙ (35) | O (33) | O (32) | O (30) | O (30) | Δ (29) | × (19) |
| | rich texture on application | ⊙ (39) | ⊙ (38) | ⊙ (39) | ⊙ (39) | ⊙ (39) | O (33) | O (34) | O (32) | O (30) |
| | melting feel on the skin | O (32) | ⊙ (38) | ⊙ (37) | O (34) | O (32) | O (32) | O (30) | × (10) | × (9) |
| | sticky feel | O (32) | ⊙ (38) | ⊙ (37) | O (34) | O (34) | ⊙ (36) | O (33) | × (10) | O (30) |

**[Table 6]**

| common additive component | |
|---|---|
| carbomer (% by mass) | 0.3 |
| glycerin (% by mass) | 5 |
| beeswax (% by mass) | 3 |
| behenyl alcohol (% by mass) | 5 |
| PEG-75 Stearate (% by mass) | 1 |
| polysorbate 80 (% by mass) | 1 |
| glyceryl stearate (% by mass) | 2 |
| Phenoxytol (% by mass) | 0.3 |
| total (% by mass) | 17.6 |

As shown in Table 5, the cosmetics of Examples 6 to 12, which contained polyolefins 1 to 5 (base material for cosmetics materials) and a large amount of common additive components (wax, etc. that are solid at room temperature), showed good stability even under harsh conditions such as temperature cycles from -20°C to 50°C assuming winter bathroom temperatures. Furthermore, the cosmetics of Examples 6 to 12 were superior in the rich texture on application and melting feel on the skin and had no sticky feel.

On the other hand, the cosmetic of Comparative Example 3 or 4 containing polyolefin C1 or C2 was poor in the temperature cycle stability, melting feel on the skin and sticky feel.

### <Example 13: Production of milky lotion>

By mixing the components according to the composition shown in Table 6 below, a milky lotion (cosmetic) is obtained.

**[Table 7]**

| Example 13: milky lotion | |
|---|---|
| | composition |
| polyolefin 2 (Example 2) | 3% |
| C13-15 Alkane | 2% |
| squalane | 2% |
| Dimethicone | 1% |
| Olea Europaea (Olive) Fruit Oil | 2% |
| ethylhexyl palmitiate | 2% |
| glycerin | 5% |
| 1,3-butylene glycol | 2% |
| Polyglyceryl-5 Stearate | 3% |
| PEG/PPG/Polybutylene Glycol-8/5/3 Glycerin | 0.5% |
| Polyquaternium-51 | 0.1% |
| sodium hyaluronate (1% aqueous solution) | 0.01% |
| dipotassium glycyrrhizinate | 1% |
| phenoxyethanol | 0.3% |
| ethylhexylglycerin | 0.1% |
| carbomer | 0.3% |
| .potassium hydroxide | appropriate amount |
| water | rest |
| total | 100% |

| | |
|---|---|
| "%" = "% by mass" | |

### <Example 14: Production of cream>

By mixing the components according to the composition shown in Table 8 below, a cream (cosmetic) is obtained.

**[Table 8]**

| Example 14: cream | |
|---|---|
| | composition |
| polyolefin 2 (Example 2) | 3% |
| C9-12 Alkane | 2% |
| squalane | 2% |
| Olea Europaea (Olive) Fruit Oil | 2% |
| ethylhexyl palmitiate | 2% |
| glycerin | 5% |
| propylene glycol | 3% |
| 1,3-butylene glycol | 2% |
| cetanol | 2% |
| behenyl alcohol | 2% |
| beeswax | 1% |
| Polyglyceryl-5 Laurate | 3% |
| PEG/PPG/Polybutylene Glycol-8/5/3 Glycerin | 0.5% |
| Polyquaternium-51 | 0.1% |
| sodium hyaluronate (1% aqueous solution) | 0.01% |
| tocopherol | 0.05% |
| niacin amide | 3% |
| phenoxyethanol | 0.2% |
| ethylhexylglycerin | 0.1% |
| citric acid | appropriate amount |
| sodium citrate | appropriate amount |
| water | rest |
| total | 100% |

| | |
|---|---|
| "%" = "% by mass" | |

### <Example 15: Production of sunscreen>

By mixing the components according to the composition shown in Table 9 below, a sunscreen (cosmetic) is obtained.

**[Table 9]**

| Example 15: sunscreen | |
|---|---|
| | composition |
| polyolefin 2 (Example 2) | 3% |
| C9-12 Alkane | 2% |
| squalane | 2% |
| Dimethicone | 1% |
| Olea Europaea (Olive) Fruit Oil | 2% |
| ethylhexyl palmitiate | 2% |
| Disteardimonium Hectorite | 2% |
| Acrylates/C10-30 Alkyl Acrylate Crosspolymer | 0.1% |
| hydrogenated polyisobutene | 5% |
| titanium oxide | 6% |
| zinc oxide | 5% |
| ethylhexyl methoxycinnamate | 2.8% |
| diethylamino hydroxybenzoyl hexyl benzoate | 2.2% |
| PEG/PPG/Polybutylene Glycol-8/5/3 Glycerin | 0.5% |
| Polyquaternium-51 | 0.1% |
| glycerin | 5% |
| 1,3-butylene glycol | 2% |
| sodium hyaluronate (1% aqueous solution) | 0.01% |
| phenoxyethanol | 0.2% |
| ethylhexylglycerin | 0.1% |
| arginine | appropriate amount |
| water | rest |
| total | 100% |

| | |
|---|---|
| "%" = "% by mass" | |

### <Example 16: Production of point makeup remover>

By mixing the components according to the composition shown in Table 10 below, a point makeup remover (cosmetic) is obtained.

**[Table 10]**

| Example 16: point makeup remover | |
|---|---|
| | composition |
| polyolefin 2 (Example 2) | 1% |
| C9-12 Alkane | 5% |
| cetyl ethylhexanoate | 15% |
| Caprylic/Capric Triglyceride | 1.5% |
| sodium chloride | 1% |
| DPG | 7% |
| glycerin | 3% |
| pentylene glycol | 1% |
| olive fruit oil | 1% |
| 1,2-hexanediol | 0.3% |
| Ethanol | 5% |
| BG | 1% |
| EDTA-2Na | 0.25% |
| tocopherol | 0.05% |
| water | rest |
| total | 100% |

| | |
|---|---|
| "%" = "% by mass" | |

### <Example 17: Production of cleansing oil>

By mixing the components according to the composition shown in Table 11 below, a cleansing oil (cosmetic) is obtained.

**[Table 11]**

| Example 17: cleansing oil | |
|---|---|
| | composition |
| polyolefin 2 (Example 2) | 2% |
| C9-12 Alkane | 18% |
| cetyl ethylhexanoate | 20% |
| squalane | 5% |
| Olea Europaea (Olive) Fruit Oil | 10% |
| Sorbeth Tetraisostearate | 15% |
| Polyglyceryl-10 Diisostearate | 6% |
| Sorbitan Oleate | 4% |
| glyceryl laurate | 3% |
| Polysorbate 20 | 7% |
| ethyl oleate | 6% |
| tocopherol | 0.05% |
| Fragrance | 0.3% |
| water | rest |
| total | 100% |

| | |
|---|---|
| "%" = "% by mass" | |

### <Example 18: Production of hair oil>

By mixing the components according to the composition shown in Table 12 below, a hair oil (cosmetic) is obtained.

**[Table 12]**

| Example 18: hair oil | |
|---|---|
| | composition |
| polyolefin 2 (Example 2) | 5% |
| C9-12 Alkane | 10% |
| C13-15 Alkane | 10% |
| Dimethicone | 65% |
| Olea Europaea (Olive) Fruit Oil | 10% |
| Aminopropyl Dimethicone | 5% |
| tocopherol | 0.05% |
| Fragrance | 0.3% |
| water | rest |
| total | 100% |

| | |
|---|---|
| "%" = "% by mass" | |

### <Example 19: Production of treatment>

By mixing the components according to the composition shown in Table 13 below, a treatment (cosmetic) is obtained.

**[Table 13]**

| Example 19: treatment | |
|---|---|
| | composition |
| polyolefin 2 (Example 2) | 5% |
| C9-12 Alkane | 2% |
| Behentrimonium Chloride | 1.5% |
| Stearamidopropyl Dimethylamine | 0.5% |
| squalane | 1% |
| Dimethicone | 5% |
| Aminopropyl Dimethicone | 1% |
| behenyl alcohol | 1% |
| cetanol | 4% |
| glycerin | 5% |
| citric acid | appropriate amount |
| sodium citrate | appropriate amount |
| phenoxyethanol | 5% |
| tocopherol | 0.05% |
| Fragrance | 0.3% |
| water | rest |
| total | 100% |

| | |
|---|---|
| "%" = "% by mass" | |

### <Example 20: Production of mascara>

By mixing the components according to the composition shown in Table 14 below, a mascara is obtained.

**[Table 14]**

| Example 20: mascara | |
|---|---|
| | composition |
| polyolefin 2 (Example 2) | 3% |
| C13-15 Alkane | 20% |
| iron oxide | 150 |
| microcrystalline wax | 10% |
| candelilla wax | 1.5% |
| Ethanol | 1% |
| talc | 1% |
| trimethylsiloxysilicate | 10% |
| Disteardimonium Hectorite | 2% |
| Dextrin Palmitate | 1% |
| Propylparaben | 0.05% |
| tocopherol | 0.05% |
| hydrogenated polyisobutene | rest |
| total | 100% |

| | |
|---|---|
| "%" = "% by mass" | |

### <Example 21: Production of liquid foundation>

By mixing the components according to the composition shown in Table 15 below, a liquid foundation (cosmetic) is obtained.

**[Table 15]**

| Example 21: liquid foundation | |
|---|---|
| | composition |
| polyolefin 2 (Example 2) | 3% |
| C13-15 Alkane | 150 |
| Dimethicone | 15% |
| beeswax | 1% |
| sodium hyaluronate | 0.01% |
| Polyquaternium-51 | 0.01% |
| BG | 5% |
| DPG | 5% |
| propanediol | 3% |
| aluminum hydroxide | appropriate amount |
| stearic acid | appropriate amount |
| tocopherol | 0.05% |
| phenoxyethanol | 0.5% |
| PEG-9 Polydimethylsiloxyethyl Dimethicone | 1% |
| talc | 4% |
| titanium oxide | 8% |
| iron oxide | 2% |
| water | rest |
| total | 100% |

| | |
|---|---|
| "%" = "% by mass" | |

### <Example 22: Production of lipstick>

By mixing the components according to the composition shown in Table 16 below, a lipstick (cosmetic) is obtained.

**[Table 16]**

| Example 22: lipstick | |
|---|---|
| | composition |
| polyolefin 2 (Example 2) | 5% |
| C13-15 Alkane | 2% |
| hydrogenated polyisobutene | rest |
| castor oil | 5% |
| jojoba oil | 5% |
| olive oil | 5% |
| Carnauba wax | 2% |
| polyethylene wax | 9% |
| silicone resin | 18% |
| dimethylpolysiloxane (SH100) | 5% |
| aluminum hydroxide | appropriate amount |
| Red No. 201 (CI 15850) | 1% |
| titanium oxide | 2% |
| pearl pigment | 5% |
| total | 100% |

| | |
|---|---|
| "%" = "% by mass" | |

### [Industrial Applicability]

The polyolefin of the present invention is useful as a plasticizer for electronic materials, a base material for cosmetics, a lubricant, or the like.

This application is based on a patent application No. 2022-066549 filed in Japan, the contents of which are incorporated in full herein.

## Claims

1. A polyolefin having a number average molecular weight of 250 to 10,000 and obtained from a linear olefin monomer having 8 to 20 carbon atoms, wherein a rate of side chains having (n-3) carbon atoms or lower than (n-3) carbon atoms is 1 to 40%, as calculated from the formula (1): rate of side chains having (n-3) carbon atoms or lower than (n-3) carbon atoms (%) = [Nx/(Nx+Ny)×2n-3]/3×100 ··· [in the formula (1),
n is an average number of carbon atoms of the olefin monomer,
Nx is an integral value of a signal at 0.75 to 0.95 ppm detected in ¹H-NMR measurement of the polyolefin, and
Ny is an integral value of a signal at 1.00 to 1.45 ppm detected in ¹H-NMR measurement of the polyolefin].

2. A method for producing the polyolefin of according to claim 1, comprising polymerizing a linear olefin monomer having 8 to 20 carbon atoms in the presence of a Lewis acid catalyst.

3. A plasticizer for electronic materials, consisting of the polyolefin according to claim 1.

4. A base material for cosmetics, consisting of the polyolefin according to claim 1.

5. A cosmetic comprising the base material for cosmetics according to claim 4 at a content of 1 to 80% by mass with respect to the whole cosmetic.

6. A lubricant consisting of the polyolefin according to claim 1.
